(19) Europäisches Patentamt
European Patent Office
Office européen des brevets

(11) Veröffentlichungsnummer: **0 268 093 B1**

(12) **EUROPÄISCHE PATENTSCHRIFT**

(45) Veröffentlichungstag der Patentschrift: **13.05.92**

(51) Int. Cl.⁵: **C07D 221/14**, A61K 31/47

(21) Anmeldenummer: **87115310.2**

(22) Anmeldetag: **20.10.87**

Die Akte enthält technische Angaben, die nach dem Eingang der Anmeldung eingereicht wurden und die nicht in dieser Patentschrift enthalten sind.

# (54) 5-Nitrobenzo[de]isochinolin-1,3-dione, ihre Herstellung und Verwendung.

(30) Priorität: **21.10.86 DE 3635711**

(43) Veröffentlichungstag der Anmeldung:
**25.05.88 Patentblatt 88/21**

(45) Bekanntmachung des Hinweises auf die Patenterteilung:
**13.05.92 Patentblatt 92/20**

(84) Benannte Vertragsstaaten:
**AT BE CH DE ES FR GB IT LI NL SE**

(56) Entgegenhaltungen:
**EP-A- 0 125 439**

(73) Patentinhaber: **KNOLL AG**
**Knollstrasse 32**
**W-6700 Ludwigshafen(DE)**

(72) Erfinder: **Brana Fernandez, Miguel, Dr.**
**Avenida de Burgos No. 20, 1o-M.**
**ES-28036 Madrid(ES)**
Erfinder: **Berlanga Castellano, José Maria, Dr.**
**Avenida de Burgos No. 20, 1o-M.**
**ES-28036 Madrid(ES)**
Erfinder: **Morán Moset, Marina, Dr,**
**Calle Gaztambide 50**
**ES-28015 Madrid(ES)**
Erfinder: **Schlick, Erich, Dr.**
**Rudolph-Wihr-Str. 4 1/2**
**W-6708 Neuhofen(DE)**
Erfinder: **Keilhauer, Gerhard, Dr.**
**Industriestrasse 20**
**WE-6701 Dannstadt-Schauernheim(DE)**

(74) Vertreter: **Karau, Wolfgang, Dr. et al**
**BASF Aktiengesellschaft Carl-Bosch-Strasse 38**
**W-6700 Ludwigshafen(DE)**

EP 0 268 093 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 5-Nitrobenzo[de]isochinoline, Verfahren zu deren Herstellung und deren Verwendung bei der Bekämpfung von Krankheiten.

Es ist bereits bekannt, daß bestimmmte Benzo[de]isochinoline tumorhemmende Eigenschaften besitzen (Arzneim. Forsch./Drug Res. 34 (II), 1243 (1984). Speziell in der EP-A 125 439 werden 5,8- disubstituierte Benzo[de]isochinolin-1,3-dione beschrieben, die u.a. turmorhemmende Eigenschaften besitzen. Sie unterscheiden sich von den erfindungsgemäßen Verbindungen darin, daß die Substituenten in 5,8-Position gleich sind, entweder jeweils $NO_2$ oder $NH_2$, während die vorliegender Verbindungen in 5-Stellung nitro- und in 8-Stellung aminosubstituiert sind. Die Wirkungen der bisher beschriebenen Benzo[de]isochinoline sind jedoch nicht in jeder Hinsicht befriedigend.

Es wurde nun gefunden, daß 5-Nitrobenzo[de]isochinolin-1,3-dione der Formel I

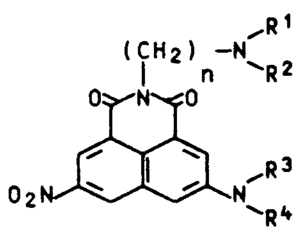

I,

worin

n die Zahl 1 oder 2 bedeutet,

$R^1$ und $R^2$ gleich oder verschieden sind und Wasserstoff, $C_1$-$C_6$-Alkyl, $C_1$-$C_6$-Hyroxyalkyl oder zusammen mit dem Stickstoffatom, an welches sie gebunden sind, Pyrrolidinyl, Morpholino, Piperidinyl oder Piperacinyl bedeuten und

$R^3$ und $R^4$ gleich oder verschieden sind und Wasserstoff, $C_1$-$C_6$-Alkyl, $C_1$-$C_6$-Acyl, $C_2$-$6_7$-Alkoxycarbonyl, Aminocarbonyl oder $C_2$-$C_7$-Alkylaminocarbonyl bedeuten,

sowie deren Salze mit physiologisch verträglichen Säuren eine bessere Wirkung bzw. ein besseres Wirkungsspektrum als tumorhemmende Substanzen besitzen.

Bevorzugt sind die Verbindungen, in denen $R^1$ und $R^2$ Methyl oder Ethyl, $R^3$ ein Wasserstoffatom oder $C_1$-$C_6$-Acyl - insbesondere Acetyl - und $R^4$ ein Wasserstoffatom darstellen.

Als physiologisch verträgliche Säuren eignen sich zur Salzbildung organische und anorganische Säuren, wie Salzsäure, Schwefelsäure, Phosphorsäure, Essigsaure, Zitronensäure, Oxalsäure, Malonsäure, Salizylsäure, Maleinsäure, Fumarsäure, Bernsteinsäure, Ascorbinsäure, Apfelsäure, Methansulfonsäure, Isethionsäure, Milchsäure, Gluconsäure, Glucuronsäure, Amidosulfonsäure, Benzoesäure, Weinsäure, Pamoasäure.

Die neuen Verbindungen können in solvatisierter Form vorliegen. Solche Formen können sich beispielsweise mit Wasser oder Ethanol bilden.

Die neuen Verbindungen werden hergestellt, indem man

a) ein 3-Nitro-1,8-naphthalsäureanhydrid der Formel II

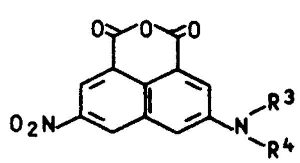

II,

worin $R^3$ und $R^4$ die angegebene Bedeutung haben, mit einem Amin der Formel III

$H_2N$-$(CH_2)$ -$NR^1R^2$ III,

worin n, $R^1$ und $R^2$ die angegebene Bedeutung haben, umsetzt oder

b) ein 5,8-Dinitrobenzo[de]isochinolin der Formel IV

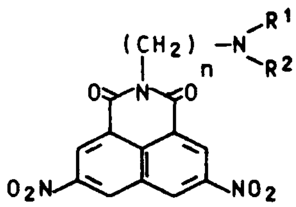

IV,

worin n, $R^1$ und $R^3$ die angegebene Bedeutung besitzen, selektiv reduziert und das so erhaltene 5-Nitro-8-amino-Derivat gegebenenfalls alkyliert bzw. acyliert

und die so erhaltenen Verbindungen gegebenenfalls in deren Salze mit physiologisch verträglichen Säuren überführt.

Die Umsetzung gemäß a) wird in einem geeigneten Lösungsmittel wie Methanol, Ethanol, Propanol oder Aceton in der Regel bei Raumtemperatur durchgeführt. Die erfindungsgemäße Verbindung scheidet sich aus dem Reaktionsgemisch ab und kann chromatographisch und/oder durch Umkristallisation gereinigt werden.

Die Reduktion gemäß b) kann mit einem Alkalimetallhydrid oder -polysulfid, Zinn(II)-chlorid oder durch katalytische Hydrierung mit der berechneten Menge Wasserstoff erfolgen. Die so erhaltenen Amine können anschließend gewünschtenfalls in an sich bekannter Weise alkyliert, acyliert oder mit Alkyl- oder Metallisocyanaten umgesetzt werden.

Die so erhaltenen Verbindungen können in an sich bekannter Weise in ihre Salze überführt werden, z.B. durch Umsetzen mit einer Säure.

Die erfindungsgemäßen Verbindungen können in üblicher Weise oral oder parenteral verabfolgt werden. Sie können in den gebräuchlichen galenischen Applikationsformen fest oder flüssig angewendet werden, z.B. als Tabletten, Filmtabletten, Kapseln, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidatien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 90 Gew.%.

Die Cytotoxizität der neuen Verbindungen wurde wie folgt bestimmt:

$5 \times 10^3$ im exponentiellen Wachstum sich befindliche humane Tumor-Zellen wurden in 96-Loch-Platten in 125 $\mu$l komplettem Wachstumsmedium (MEM mit Earle's Salzen + 10 % FCS, Flow Laboratories, Meckenheim, FRC) ausplattiert und über Nacht bei 37°C in Gegenwart von 5 % $CO_2$ in einer Wasserdampf-gesättigten Atmosphäre inkubiert. Die Substanzzugabe erfolgte am nächsten Tag in 25 $\mu$l komplettem Kulturmedium pro Kulturloch. Die Endkonzentration betrug jeweils $10^{-4}$ Mol Protein pro Loch; titriert wurde seriell 2-fach mit einer Doppelbestimmung. Folgende Kontrollen wurden auf jeder Kulturplatte mitangelegt: a) nur Kulturmedium; b) Zellen mit Kulturmedium, aber ohne Wirksubstanz; c) ein titrierter Vergleichssubstanz-Standard mit bekann ter biologischer Aktivität. Nach einer weiteren Inkubation von 72 h bei den oben angegebenen Bedingungen wurden die überlebenden Zellen mit einer Kristallviolettlösung (15 g Kristallviolett, 7 g NaCl, 646 ml Ethanol, 172,8 ml 37 %iges Formaldehyd auf 2 l mit $H_2O$ aufgefüllt) angefärbt. Dazu wurden nach dem Abschlagen des Kulturmediums die Zellen für 20 min mit 50 $\mu$l der Färbelösung bei Raumtemperatur gefärbt. Die Kulturplatten wurden danach mit Wasser gewaschen, um nicht zellgebundene Farbstoffanteile zu entfernen. Der zellgebundene Farbstoff wurde nach Zugabe von 100 $\mu$l Meßlösung (50 % Ethanol, 0,1 % Essigsäure) bei 540 nm mit Hilfe eines Titertek Multiscan MCC/340 (Flow Laboratories, Meckenheim) photometrisch bestimmt. Angegeben wurden die Konzentrationen an Substanz, die eine 50 %ige Lyse der Zellen im Vergleich zu der unbehandelten Zellkontrolle ergeben (gemessen über die Reduktion der Absorption).

In diesem Test zeigten die neuen Verbindungen - insbesondere die Substanz des Beispiels 1 - eine gute Wirkung.

Beispiel 1

1,16 g (0,01 Mol) N,N-Diethylethylendiamin wurden zu 2,58 g (0,01 Mol) 6-Amino-3-nitro-1,8-naphthalsäureanyhdrid in 20 ml Ethanol gegeben. Die Mischung wurde 6 h gerührt und über Nacht stehen gelassen. Das ausgeschiedene Produkt wurde abgesaugt, an Silicagel chromatographiert ($CHCl_3$ : $CH_3OH$ = 7 : 10) und aus Methanol/Wasser umkristallisiert. Man erhielt 8-Amino-2-[2-(diethylamino)-ethyl]-5-nitrobenzo[de]-isochinolin-1,3-dion Fp. 174 °C (Toluol).

Beispiel 2

4,7 g Zinn(II)-chlorid wurden in 8,7 ml heißem Eisessig aufgeschlämmt und unter Einleiten von Chlorwasserstoff auf 0 °C abgekühlt. Die so erhaltene Lösung wurde zu einer Lösung von 2 g (0,005 Mol) 2-[2-(Dimethylamino)-ethyl]-5,8-dinitriobenzo[de]isochinolin-1,3-dion in heißem Eisessig gegeben und 45 min bei einer Temperatur von unter 30 °C gerührt. Nach Stehen über Nacht wurde das breiige Produkt mit 1 ml Wasser bei 70 °C versetzt. Aus dem Reaktionsgemisch wurde im Vakuum der größte Teil des Eisessigs abgezogen. Der Rückstand wurde mit Wasser gewaschen und mit 28 ml 20 %iger Natronlauge versetzt, wobei die Temperatur 25 °C nicht überstieg. Das so erhaltene feste Produkt wurde abfiltriert und so lange mit siedender 10 %iger Salzsäure gewaschen, bis aus der Waschflüssigkeit nach Zugabe von Ammoniak kein Niederschlag mehr ausfiel. Das 8-Amino-2-[2-(dimethylamino)-ethyl]-5-nitrobenzo[de]isochinolin-1,3-dion wurde an Kieselgel chromatographiert ($CHCl_3$ : $CH_3OH$ = 7 : 10) und aus Toluol umkristallisiert, Fp. 266-268 °C.

Analog Beispiel 1 und 2 wurden hergestellt:

3. 8-Amino-5-nitro-2-[2-(pyrrolidinyl)-ethyl]-benzo[de]-isochinolin-1,3-dion, Fp. 320 °C (Toluol).
4. 8-Amino-5-nitro-2-[2-(piperidinyl)-ethyl]-benzo[de]-isochinolin-1,3-dion, Fp. 232-234 °C (Toluol).
5. 8-Amino-2-[3-[di-(2-hydroxyethyl)-amino]-propyl]-5-nitrobenzo[de]isochinolin-1,3-dion, Fp. 151-152 °C (Toluol).
6. 8-Amino-2-[2-(2-hydroxyethyl)-aminoethyl]-5-nitrobenzo[de]-isochinolin-1,3-dion, Fp. > 280 °C (DMF-Wasser).
7. 8-Amino-2-[2-[di-(2-hydroxyethyl)-amino]-ethyl]-5-nitrobenzo[de]isochinolin-1,3-dion, Fp. > 280 °C (DMF-Wasser).
8. 8-Amino-2-[2-(morpholino)-ethyl]-5-nitrobenzo[de]-isochinolin-1,3-dion, Fp. 234 °C (Toluol).
9. 8-Ethoxycarbonylamino-2-[2-(dimethylamino)-ethyl]-5-nitrobenzo[de]isochinolin-1,3-dion, Fp. 320 °C (Eisessig).

Beispiel 10

3,54 g (0,01 Mol) 8-Amino-5-nitro-2-[2-(pyrrolidinyl)-ethyl]-benzo[de]isochinolin-1,3-dion (vgl. Beispiel 3) und 3,05 g (0,05 Mol) Ethylisocyanat wurden in 50 ml Benzol 2 h unter Rückfluß gekocht. Das ausfallende 8-Ethylaminocarbonylamino-5-nitro-2-[2-(pyrrolidinyl)-ethyl]benzo[de]-i ochinolin-1,3-dion wurde abfiltriert und aus Ethanol umkristallisiert, Fp. 234 °C.

Analog Beispiel 10 wurde hergestellt:

11. 8-Ethylaminocarbonylamino-5-nitro-2-(2-dimethylaminoethyl)-ben -isochinolin-1,3-dion, Fp. 166 °C (Essigsäureethylester).

Beispiel 12

Eine Mischung aus 3,28 g (0,01 Mol) 8-Amino-2-[2-(dimethylamino)-ethyl]-5-nitrobenzo[de]-isochinolin-1,3-dion (vgl. Beispiel 2), 10 ml Acetylchlorid und 20 ml Essigsäureanhydrid wurde 1 h unter Rühren unter Rückfluß gekocht. Nach dem Abkühlen wurde das Reaktionsgemisch auf 50 g Eis gegossen und mit Natriumhydrogencarbonat neutralisiert. Das ausgefallene 8-Acetylamino-2-[2-(dimethylamino)-ethyl]-5-nitrobenzo[de]-isochinolin-,3-dion wurde abfiltriert und aus DMF/Wasser umkristallisiert, Fp. 320 °C (Eisessig).

## Patentansprüche

**Patentanspruch für folgende Vertragsstaaten : BE, CH, DE, ES, FR, GB, IT, LI, NL, SE**

1. 5-Nitrobenzo[de]isochinolin-1,3-dione der Formel I

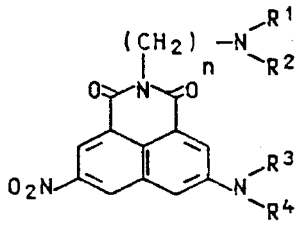

I,

worin
n die Zahl 1 oder 2 bedeutet,
$R^1$ und $R^2$ gleich oder verschieden sind und Wasserstoff, $6_1$-$C_6$-Alkyl, $C_1$-$C_6$-Hydroxyalkyl oder zusammen mit dem Stickstoffatom, an welches sie gebunden sind, Pyrrolidinyl, Morpholino, Piperidinyl oder Piperacinyi bedeuten und
$R^3$ und $R^4$ gleich oder verschieden sind und Wasserstoff, $C_1$-$C_6$-Alkyl, $C_1$-$C_6$-Acyl, $C_2$-$C_7$-Alkoxycarbonyl, Aminocarbonyl oder $C_2$-$C_7$-Alkylaminocarbonyl bedeuten,
sowie deren Salze mit physiologisch verträglichen Säuren.

2. Verbindungen gemaß Anspruch 1 , dadurch gekennzeichnet, daß $R^1$ und $R^2$ Methyl oder Ethyl und $R^3$ und $R^4$ Wasserstoffatome darstellen.

3. 8-Amino-2-[2-(dimethylamino)-ethyl)-5-nitrobenzo[de]isochinolin-,3-dion.

4. Verfahren zur Herstellung der Verbindungen I gemäß Anspruch 1, dadurch gekennzeichnet, daß man
   a) ein 3-Nitro-1 ,8-naphthalsäureanhydrid der Formel II

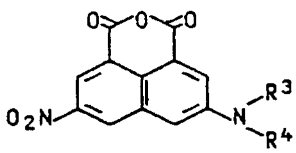

II,

mit einem Amin der Formel III

$H_2N$-$(CH_2)_n$-$NR^1R^2$  III,

umsetzt oder

b) ein 5,8-Dinitrobenzo[de]isochinolin der Formel IV

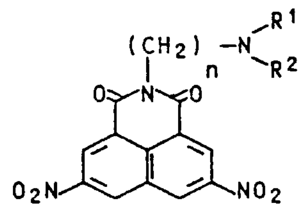

IV,

selektiv reduziert und das so erhaltene 5-Nitro-8-amino-Derivat gegebenenfalls alkyliert bzw. acyliert und die so erhaltenen Verbindungen gegebenenfalls in deren Salze mit physiologisch verträglichen Säuren überführt.

**5.** Verfahren gemäß Anspruch 4, dadurch gekennzeichnet, daß man 5-Nitrobenzo[de]isochinolin-1,3-dione der Formel I gemäß Anspruch 1, worin $R^1$ und $R^2$ Methyl oder Ethyl und $R^3$ und $R^4$ Wasserstoffatome darstellen, herstellt.

**6.** Verfahren gemaß Anspruch 4, dadurch gekennzeichnet, daß man 8-Amino-2-[2-(dimethylamino)-ethyl]-5-nitrobenzo[de]isochinolin-1,3-dion herstellt.

**Patentansprüche für folgenden Vertragsstaat : AT**

**1.** Verfahren zur Herstellung der 5-Nitrobenzo[de]isochinolin-1,3-dione der Formel I

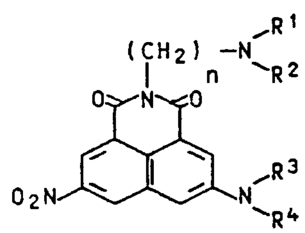

I,

worin

n die Zahl 1 oder 2 bedeutet,

$R^1$ und $R^2$ gleich oder verschieden sind und Wasserstoff, $C_1$-$C_6$-Alkyl, $C_1$-$C_6$-Hydroxyalkyl oder zusammen mit dem Stickstoffatom, an welches sie gebunden sind, Pyrrolidinyl, Morpholino, Piperidinyl oder Piperacinyl bedeuten und

$R^3$ und $R^4$ gleich oder verschieden sind und Wasserstoff, $C_1$-$C_6$-Alkyl, $C_1$-$C_6$-Acyl, $C_2$-$C_7$-Alkoxycarbonyl, Aminocarbonyl oder $C_2$-$C_7$-Alkylaminocarbonyl bedeuten,

sowie deren Salze mit physiologisch verträglichen Säuren, dadurch gekennzeichnet, daß man

a) ein 3-Nitro-1,8 -naphthalsäureanhydrid der Formel II

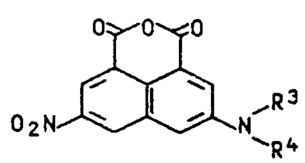

II

mit einem Amin der Formel III

$H_2N$-$(CH_2)_n$ -$NR^1R^2$ III

umsetzt oder

b) ein 5,8-Dinitrobenzo[de]isochinolin der Formel IV

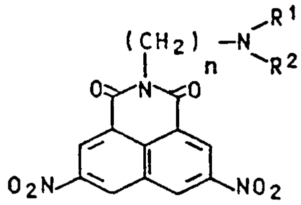

IV

selektiv reduziert und das so erhaltene 5-Nitro-8-amino-Derivat gegebenenfalls alkyliert bzw. acyliert und die so erhaltenen Verbindungen gegebenenfalls in deren Salze mit physiologisch verträglichen Säuren überführt.

**2.** Verfahren gemäß Anspruch 1, dadurch gekennzeichnet, daß man 5-Nitrobenzo[de]isochinolin-1,3-dione der Formel I gemäß Anspruch 1, worin $R^1$ und $R^2$ Methyl oder Ethyl und $R^3$ und $R^4$ Wasserstoffatome darstellen, herstellt.

**3.** Verfahren gemäß Anspruch 1, dadurch gekennzeichnet, daß man 8-Amino-2-[2-(dimethylamino)-ethyl]-5-nitrobenzo[de]isochinolin-1,3-dion herstellt.

## Claims

### Claims for the following Contracting States : BE, CH, DE, ES, FR, GB, IT, LI, NL, SE

**1.** A 5-nitrobenzo[de]isoquinoline-1,3-dione of the formula I

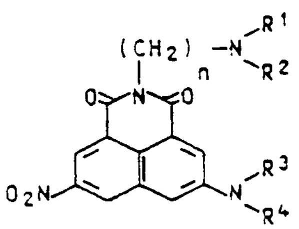

I

where n is 1 or 2, $R^1$ and $R^2$ are identical or different and are each hydrogen, $C_1$-$C_6$-alkyl or $C_1$-$C_6$-hydroxyalkyl, or, together with the nitrogen atom to which they are bonded, form pyrrolidinyl, morpholino, piperidinyl or piperacinyl and $R^3$ and $R^4$ are identical or different and are each hydrogen, $C_1$-$C_6$-alkyl, $C_1$-$C_6$-acyl, $C_2$-$C_7$-alkoxy-carbonyl, aminocarbonyl or $C_2$-$C_7$-alkylaminocarbonyl, and its salts with physiologically tolerated acids.

**2.** A compound as claimed in claim 1, wherein $R^1$ and $R^2$ are each methyl or ethyl and $R^3$ and $R^4$ are each hydrogen.

**3.** An 8-amino-2-[2-(dimethylamino)-ethyl]-5-nitrobenzo[de]isoquinoline-1,3-dione.

**4.** A process for the preparation of a compound I as claimed in claim 1, wherein

a) a 3-nitro-1,8-naphthalic anhydride of the formula II

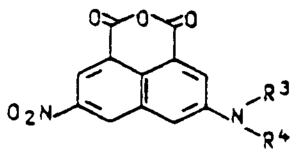

II

is reacted with an amine of the formula III

$H_2N-(CH_2)_n-NR^1R^2$ III

or

b) a 5,8-dinitrobenzo[de]isoquinoline of the formula IV

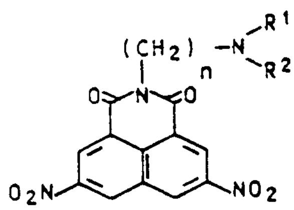

IV

is selectively reduced, and the resulting 5-nitro-8-amino derivative is, if required, alkylated or acylated,

and the resulting compound is, if required, converted to its salts with physiologically tolerated acids.

**5.** A process as claimed in claim 4, wherein a 5-nitrobenzo[de]isoquinoline-1,3-dione of the formula I as claimed in claim 1, where $R^1$ and $R^2$ are each methyl or ethyl and $R^3$ and $R^4$ are each hydrogen, is prepared.

**6.** A process as claimed in claim 4, wherein 8-amino-2-[2-(dimethylamino)-ethyl]-5-nitronzo[de]-isoquinoline-1,3-dione is prepared.

**Claims for the following Contracting State : AT**

**1.** A process for the preparation of a 5-nitrobenzo[de]isoquinoline-1,3-dione of the formula I

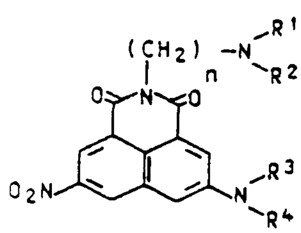

I

where n is 1 or 2, $R^1$ and $R^2$ are identical or different and are each hydrogen, $C_1$-$C_6$-alkyl or $C_1$-$C_6$-hydroxyalkyl, or, together with the nitrogen atom to which they are bonded, form pyrrolidinyl, morpholino, piperidinyl or piperacinyl and $R^3$ and $R^4$ are identical or different and are each hydrogen, $C_1$-$C_6$-alkyl, $C_1$-$C_6$-acyl, $C_2$-$C_7$-alkoxycarbonyl, aminocarbonyl or $C_2$-$C_7$-alkylaminocarbonyl, and its salts with physiologically tolerated acids, wherein

a) a 3-nitro-1,8-naphthalic anhydride of the formula II

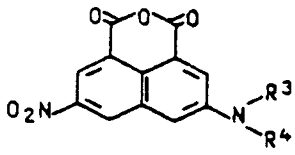

II

is reacted with an amine of the formula III

$H_2N-(CH_2)_n-NR^1R^2$ III

or

b) a 5,8-dinitrobenzo[de]isoquinoline of the formula IV

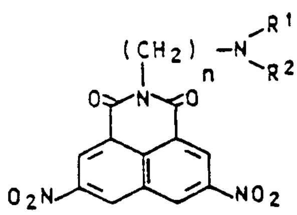

IV

is selectively reduced, and the resulting 5-nitro-8-amino derivative is, if required, alkylated or acylated,

and the resulting compound is, if required, converted to its salts with physiologically tolerated acids.

**2.** A process as claimed in claim 1, wherein a 5-nitrobenzo[de]isoquinoline-1,3-dione of the formula I as claimed in claim 1, where $R^1$ and $R^2$ are each methyl or ethyl and $R^3$ and $R^4$ are each hydrogen, is prepared.

**3.** A process as claimed in claim 1, wherein 8-amino-2-[2-(dimethylamino)-ethyl]-5-nitrobenzo[de]-isoquinoline-1,3-dione is prepared.

**Revendications**

**Revendications pour les Etats contractants suivants : BE, CH, DE, ES, FR, GB, IT, LI, NL, SE**

**1.** 5-nitrobenzo [de] isoquinoléine-1,3-diones de formule I

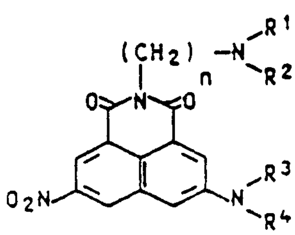

I,

dans laquelle

n est égal à 1 ou 2,

$R^1$ et $R^2$, ayant des significations identiques ou différentes, représentent chacun l'hydrogène, un groupe alkyle en C 1-C 6, hydroxyalkyle en C 1-C 6, ou forment ensemble et avec l'atome d'azote auquel ils sont reliés un cycle pyrrolidinyle, morpholino, pipéridinyle, et

$R^3$ et $R^4$, ayant des significations identiques ou différentes, représentent chacun l'hydrogène, un groupe alkyle en C 1-C 6, acyle en C 1-C 6, alcoxycarbonyle en C 2-C 7, aminocarbonyle ou alkylaminocarbonyle en C 2-C 7,
et leurs sels d'acides acceptables pour l'usage pharmaceutique.

2. Composés selon la revendication 1, caractérisés en ce que $R^1$ et $R^2$ représentent des groupes méthyle ou éthyle et $R^3$ et $R^4$ des atomes d'hydrogène.

3. La 8-amino-2- [ 2-(diméthylamino)-éthyl] -5-nitrobenzo [de] isoquinoléine-1,3-dione.

4. Procédé de préparation des composés I de la revendication 1, caractérisé en ce que :
a) on fait réagir un anhydride 3-nitro-1,8-naphtalique de formule II

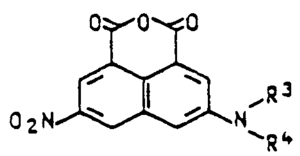

II,

avec une amine de formule III

$H_2N-(CH_2)_n-NR^1R^2$ III

ou bien
b) on soumet une 5,8-dinitrobenzo [de] isoquinoléine de formule IV

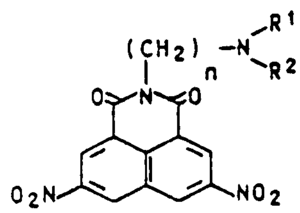

IV,

à réduction sélective et le cas échéant on soumet le dérivé 5-nitro-8-aminé ainsi obtenu à alkylation ou acylation,
et le cas échéant on convertit les composés obtenus en leurs sels d'acides acceptables pour l'usage pharmaceutique.

5. Procédé selon la revendication 4, caractérisé en ce que l'on prépare des 5-nitrobenzo de isoquinoléine-1,3-diones de formule I de la revendication 1 dans laquelle $R^1$ et $R^2$ représentent des groupes méthyle ou éthyle et $R^3$ et $R^4$ des atomes d'hydrogène.

6. Procédé selon la revendication 4, caractérisé en ce que l'on prépare la 8-amino-2- [2-(diméthylamino)-éthyl] -5-nitrobenzo de isoquinoléine-1,3-dione.

**Revendications pour l'Etat contractant suivant : AT**

**1.** Procédé de préparation des 5-nitrobenzo [de] isoquinoléine-1,3-diones de formule I

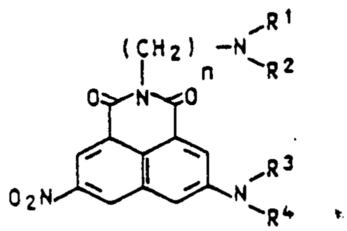

I,

dans laquelle
n est égal à 1 ou 2,
$R^1$ et $R^2_1$ ayant des significations identiques ou différentes, représentent chacun l'hydrogène, un groupe alkyle en C 1-C 6, hydroxyalkyle en C 1-C 6 ou forment ensemble et avec l'atome d'azote auquel ils sont reliés un cycle pyrrolidinyle, morpholino, pipéridinyle ou pipéracinyle, et
$R^3$ et $R^4$, ayant des significations identiques ou différentes, représentent chacun l'hydrogène, un groupe alkyle en C 1-C 6, acyle en C 1-C 6, alcoxycarbonyle en C 2-C 7, aminocarbonyle ou alkylaminocarbonyle en C 2-C 7,
et de leurs sels d'acides acceptables pour l'usage pharmaceutique, caractérisé en ce que :

a) on fait réagir un anhydride 3-nitro-1,8-naphtalique de formule II

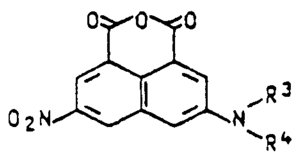

II

avec une amine de foule III

$H_2N-(CH_2)_n-NR^1R^2$ III

ou bien

b) on soumet une 5,8-dinitrobenzo [de] isoquinoléine de fomule IV

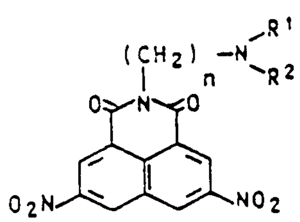

IV

à réduction sélective et le cas échéant on soumet le dérivé 5-nitro-8-aminé ainsi obtenu à alkylation ou acylation,

et le cas échéant, on convertit les composés obtenus en leurs sels d'acides acceptables pour l'usage phamaceutique.

**2.** Procédé selon la revendication 1, caractérisé en ce que l'on prépare des 5-nitrobenzo de isoguinoléine-1,3-diones de formule I de la revendication 1 dans laquelle $R^1$ et $R^2$ représentent des groupes méthyle ou éthyle et $R^3$ et $R^4$ des atomes d'hydrogène.

3. Procédé selon la revendication 1, caractérisé en ce que l'on prépare la 8-amino-2- [2-(dimétylamino)-éthyl] -5-nitrobenzo de isoquinoléine-1,3-dione.